# EUROPEAN PATENT SPECIFICATION

(11) **EP 1 499 887 B1**
(45) Date of publication and mention of the grant of the patent: **05.08.2009**
(21) Application number: 03726329.0
(22) Date of filing: 16.04.2003
(51) Int. Cl.: G01N 33/53, C07K 16/10

(54) **UNIVERSAL LIBRARIES FOR IMMUNOGLOBULINS**
UNIVERSELLE BANKEN FÜR IMMUNOGLOBULINE
BIBLIOTHEQUES UNIVERSELLES POUR IMMUNOGLOBULINES

(30) Priority: 17.04.2002 US 373558 P
(43) Date of publication of application: 26.01.2005
(73) Proprietor: Bioren, Inc., San Carlos, CA 94070 (US)
(72) Inventor: Crea, Roberto, San Mateo, California 94402 (US)
(74) Representative: Ford, Timothy James
(86) International application number: PCT/US2003/011936
(87) International publication number: WO 2003/088911

(56) References cited:
- WO-A-91/15581
- US-A- 5 667 988
- US-A- 5 798 208
- US-A- 5 852 186
- US-B1- 6 537 776
- RAJPAL ARVIND ET AL: "A general method for greatly improving the affinity of antibodies by using combinatorial libraries" PROCEEDINGS OF THE NATIONAL ACADEMY OF SCIENCES OF THE UNITED STATES OF AMERICA, vol. 102, no. 24, June 2005 (2005-06), pages 8466-8471, XP002392777 ISSN: 0027-8424
- CRAMERI A. ET AL.: 'Construction and evolution of antibody-phage libraries by DNA shuffling' NATURE MEDICINE vol. 2, no. 1, January 1996, pages 100 - 102, XP002916917
- GERSTNER R.B. ET AL.: 'Sequence plasticity in the antigen-binding site of a therapeutic anti-HER 2 antibody' JOURNAL OF MOLECULAR BIOLOGY vol. 321, 2002, pages 851 - 862, XP002972421

## Description

### BACKGROUND OF THE INVENTION

Mutagenesis is a powerful tool in the study of protein structure and function. Mutations can be made in the nucleotide sequence of a cloned gene encoding a protein of interest and the modified gene can be expressed to produce mutants of the protein. By comparing the properties of a wild-type protein and the mutants generated, it is often possible to identify individual amino acids or domains of amino acids that are essential for the structural integrity and/or biochemical function of the protein, such as its binding and/or catalytic activity. The number of mutants that can be generated from a single protein, however, renders it difficult to select mutants that will be informative or have a desired property, even if the selected mutants which encompass mutations solely in specific, putatively important regions of a protein (e.g., regions at or around the active site of a protein). For example, the substitution, deletion or insertion of a particular amino acid may have a local or global effect on the protein. A need remains for a means to assess the effects of mutagenesis of a protein systematically.

WO 91/15581 discloses the use of walkthrough mutagenesis to generate libraries of proteins, such as antibodies. US 5798208 also discloses the generation of libraries of mutant proteins using walkthrough mutagenesis. US 5852186 discloses the generation of hierarchial and random combinatorial libraries of rearranged immunoglobulin genes from peripheral blood lymphocytes of health HIV infected patients. Crameri et al (Nature, Medicine, 2(1) 1996, 100-102) discloses the use of DNA shuffling for the construction of naive human antibody phage libraries followed by the evolution of antibody sequences specific for human receptors. US 5667988 discloses methods for producing antibody libraries, and particularly for increasing antibody library diversity by inducing mutagenesis within the CDR regions of immunoglobulin heavy or light chains that are displayed on the surface of filamentous phage particles comprising the library. US 6537776 discloses the use of site-saturation mutagenesis to produce a library of mutagenized progeny polynucleotides. Gerstner et al (J. Mol. Biol. (2002) 321, 851-862) discloses anti-Her2 antibody hu4D5 phage libraries.

### SUMMARY OF THE INVENTION

The invention is drawn to libraries for an immunoglobulin of interest. The libraries, based on a prototype immunoglobulin of interest, can be generated by walk-through mutagenesis of the prototype immunoglobulin. In one embodiment, a single predetermined amino acid library of the invention comprises mutated immunoglobulins of interest in which a single predetermined amino acid has been substituted in one or more positions in one or more complementarity-determining regions of the immunoglobulin of interest; the library comprises a series of subset libraries, including: a) one subset library containing the prototype immunoglobulin of interest; b) six subset libraries (one subset library for each of the six complementarity-determining regions of the immunoglobulin of interest) containing mutated immunoglobulins in which the predetermined amino acid has been substituted in one or more positions in only one of the six complementarity-determining regions of the immunoglobulin; c) 15 subset libraries (one subset library for each of the possible combinations of two of the six complementarity-determining regions) containing mutated immunoglobulins in which the predetermined amino acid has been substituted in one or more positions in two of the six complementarity-determining regions; d) 20 subset libraries (one subset library for each of the possible combinations of three of the six complementarity-determining regions) containing mutated immunoglobulins in which the predetermined amino acid has been substituted in one or more positions in three of the six complementarity-determining regions; e) 15 subset libraries (one subset library for each of the possible combinations of four of the six complementarity-determining regions) containing mutated immunoglobulins in which the predetermined amino acid has been substituted in one or more positions in four of the six complementarity-determining regions; f) six subset libraries (one subset library for each of the possible combinations of five of the six complementarity-determining regions) containing mutated immunoglobulins in which the predetermined amino acid has been substituted in one or more positions in five of the six complementarity-determining regions; and g) one subset library comprising mutated immunoglobulins in which the predetermined amino acid has been substituted in one or more positions in all of the six complementarity-determining regions. Each subset library that contains mutated immunoglobulins contains mutated immunoglobulins in which the predetermined amino acid is present at least once at every position in the complementarity-determining region into which the predetermined amino acid has been introduced.

The predetermined amino acids are selected from the 20 naturally-occurring amino acids. The immunoglobulin of interest can be a whole immunoglobulin, or an Fab fragment of an immunoglobulin, or a single chain immunoglobulin. The immunoglobulin of interest can be any of the five types of immunoglobulins (IgG, IgM, IgA, IgD, or IgE). In one embodiment, the immunoglobulin of interest is a catalytic antibody.

The invention further relates to a universal library for a prototype immunoglobulin of interest, in which the universal library comprises 20 "single predetermined amino acid" libraries as described above, one for each of the 20 naturally-occurring amino acids. The invention additionally relates to libraries of nucleic acids encoding the single predetermined amino acid libraries as well as libraries of nucleic acids encoding the universal libraries.

The libraries described herein contain easily-identified mutated immunoglobulins that allow systematic analysis of the binding regions of the prototype immunoglobulin of interest, and also of the role of each particular preselected amino acid on the activity of the binding regions. The libraries allow generation of specific information on the particular mutations that alter interaction of the immunoglobulin of interest with its antigen, including multiple interactions by amino acids in the varying complementarity-determining regions, while at the same time avoiding problems relating to analysis of mutations generated by random mutagenesis.

### BRIEF DESCRIPTION OF THE DRAWINGS

FIG.. 1A-1B depict the complete sequence of GP-120 single chain FV, both the nucleic acid sequence (SEQ ID NO:1) and the encoded amino acid sequence (SEQ ID NO:2).
FIG. 2 depicts the overall assembly scheme for the GP-120 scFV gene shown in Fig. 1A-1B.
FIG. 3 summarizes the scFV gene libraries obtained by the methods of the invention, and the number of gene variants produced for each individual library.
FIG. 4 is a Table depicting oligonucleotide pools for use in the assembly scheme shown in FIG.2.
FIG. 5A-5B illustrate examples of oligonucleotides pools designed to introduce three (3) targeted amino acid, SER, HIS and ASP, in individual CDRs of the Fv, in a number of possible combinations. The pool sequences are given using the IUPAC nomenclature of mixed bases, shown in bold capital letters, R= A or G, Y=C or T,M=A or C,K=G or T,S=C or G,W=A or T;H=A or C or T,B=C or GorT,V=AorCorG,D=AorGorT.
FIG. 6 illustrates the strategy adopted for VL and VH gene assembly in order to generate libraries of GP-120 scFV in which three (3) CDR regions out of the six, were contemporaneously mutagenized to produce the presence of selected individual amino acids (Ser, His and Asp) in a number (8) of different combinations (L1 to L8).
FIG. 7A-7B illustrate 20 individual oligonucleotide pools, each corresponding to one of the 20 natural amino acids, for the first VL region (the first of 6 CDR regions).
FIG. 8A-8B illustrate 20 individual oligonucleotide pools, each corresponding to one of the 20 natural amino acids, for the second VL region (the second of 6 CDR regions).
FIG. 9A-9B illustrate 20 individual oligonucleotide pools, each corresponding to one of the 20 natural amino acids, for the third VL region (the third of 6 CDR regions).
FIG. 10A-10B illustrate 20 individual oligonucleotide pools, each corresponding to one of the 20 natural amino acids, for the first VH region (the fourth of 6 CDR regions).
FIG. 11A-11D illustrate 20 individual oligonucleotide pools, each corresponding to one of the 20 natural amino acids, for the second VH region (the fifth of 6 CDR regions).
FIG. 12A-12B illustrates 20 individual oligonucleotide pools, each corresponding to one of the 20 natural amino acids, for the third VH region (the sixth of 6 CDR regions).
FIG. 13A-13D show the grouping of the CDR pools for individual amino acids.

### DETAILED DESCRIPTION OF THE INVENTION

The present invention relates to libraries of immunoglobulins of interest, including libraries containing nucleic acids encoding immunoglobulins, and libraries containing immunoglobulins themselves. An "immunoglobulin," as used herein, is an antibody protein that is generated in response to, and that binds to, a specific antigen. There are five known classes, or types, of immunoglobulins: IgG, IgM, IgA, IgD and IgE (see, e.g., Dictionary of Cell and Molecular Biology, Third Edition). The basic form of an immunoglobulin is the IgG form: it includes two identical heavy chains (H) and two identical light chains (L), held together by disulfide bonds in the shape of a "Y." Heavy chains comprise four domains, including three constant domains (C_{H}) and a variable region (V_{H}). The light chains have a constant region (C_{L}) and a one variable region (V_{L}).

Each heavy-chain variable region and each light-chain variable region includes three hypervariable loops, also called complementarity-determining regions (CDRs). The antigen-binding site (Fv) region (also referred to as the "binding pocket") includes these six hypervariable (CDR) loops (three in the immunoglobulin heavy chain variable region (V_{H}) and three in the light chain variable region (V_{L})). The residues in the CDRs vary from one immunoglobulin molecule to the next, imparting antigen specificity to each antibody.

A brief description of each class of immunoglobulin follows.

### Immunoglobulin G (IgG)

IgG is the classical immunoglobulin class; IgG have a molecular weight of approximately 150 kD. As indicated above, IgG are composed of two identical light and two identical heavy chains. The IgG molecule can be proteolytically broken down into two Fab fragments and an Fc fragment. The Fabs include the antigen binding sites (the variable regions of both the light and heavy chains), the constant region of the light chain, and one of the three constant regions of the heavy chain. The Fc region consists of the remaining constant regions of the heavy chains; it contains cell-binding and complement-binding sites.

### Immunoglobulin M (IgM)

An IgM molecule (molecular weight of approximately 970 kD) is built up from five IgG type monomers joined together, with the assistance of J chains, to form a cyclic pentamer. IgM binds complement; a single IgM molecule bound to a cell surface can lyse that cell. IgM is usually produced first in an immune response before IgG.

### Immunoglobulin A (IgA)

IgA are a class of immunoglobulin found in external secretions and in serum of mammals. In secretions, IgA are found as dimers of IgG type monomers (dimers having a molecular weight of approximately 400 kD) joined by a short J-chain and linked to a secretory piece or transport piece; inn serum, they are found as monomers (molecular weight of approximately170 kD). IgAs are the main means of providing local immunity against infections in the gut or respiratory tract.

### Immunoglobulin D (IgD)

IgD (molecular weight of approximately 184 kD) is present at a low level in serum, but is a major immunoglobulin on the surface of B-lymphocytes where it may play a role in antigen recognition. Its structure resembles that of IgG but the heavy chains are of the δ type.

### Immunoglobulin E (IgE)

IgE (molecular weight of approximately 188 kD) are associated with immediate-type hypersensitivity reactions and helminth infections. They are present in very low amounts in serum and mostly bound to mast cells and basophils that have an IgE-specific Fc-receptor (Fc ε R). IgE has a high carbohydrate content and is also present in external secretions. The heavy chain is of the ε -type.

In a preferred embodiment, the immunoglobulin of interest is an immunoglobulin of class IgG. As used herein, the term "immunoglobulin of interest" can refer to an intact immunoglobulin (i.e., an immunoglobulin containing two complete heavy chains and two complete light chains). Alternatively, an immunoglobulin of interest can also refer to a portion of an immunoglobulin (i.e., an immunoglobulin containing less than the two complete heavy chains and two complete light chains), in which the portion contains the variable regions (e.g., an Fab fragment, or an Fv fragment) of an immunoglobulin. In another embodiment, the immunoglobulin of interest can also be a "single stranded" or "single chain" immunoglobulin containing, for example, a single heavy chain and a single light chain joined by linker regions, or a single chain Fv fragment. In one embodiment, for example, an immunoglobulin of interest can be prepared which includes the three variable regions of the light chain linked (e.g., with linker regions) to the three variable regions of the heavy chain, forming a single chain Fv immunoglobulin. If desired, the immunoglobulin of interest can be coupled to a larger molecule. In one embodiment, it can be coupled to a protein, such as an enzyme, toxin or cytokine. For example, proteolytic enzymes could be coupled to the immunoglobulin molecules for directing the enzymatic activity towards specific proteins, such as Fibrin for thrombolytic application, or viral coat protein and RNA for anti-viral therapy. Toxins coupled to immunoglobulins can be directed towards cancer cells (see, e.g., Antibody Engineering. R. Konterman, S. Dubel (Eds.). Springer Lab manual. Spriger-Verlag. Berlin, Heidelberg (2001), Chapter 41." Stabilization Strategies and Application of recombinant Fvs and Fv Fusion proteins". By U. Brinkmann, pp. 593-615*. et al.)* and cytokines (IL2, etc) for anti-inflammatory application, etc.

The immunoglobulin of interest can be from any species that generates antibodies, preferably a mammal, and particularly a human; alternatively, the immunoglobulin of interest can be a chimeric antibody or a "consensus" or canonic structure generated from amino acid data banks for antibodies (see, e.g., Kabat et al., J Immunol 1991 Sep 1;147(5):1709-19). The immunoglobulin of interest can be a wild-type immunoglobulin (e.g., one that is isolated or can be isolated from an organism, such as an immunoglobulin that can be found in an appropriate physiological sample (e.g., blood, serum, etc.) from a mammal, particularly a human). Alternatively, the immunoglobulin of interest can be a modified immunoglobulin (e.g., an previously wild-type immunoglobulin, into which alterations have been introduced into one or more variable regions and/or constant regions). In another embodiment, the immunoglobulin of interest can be a synthetic immunoglobulin (e.g., prepared by recombinant DNA methods, rather than isolated from an organism). In one preferred embodiment, the immunoglobulin of interest is a human immunoglobulin.

In one embodiment of the invention, the immunoglobulin of interest is a catalytic antibody. An immunoglobulin can be made catalytic, or the catalytic activity can be enhanced, by the introduction of suitable amino acids into the binding site of the immunoglobulin's variable region (Fv region) in the methods described herein. For instance, catalytic triads modeled after serine proteases can be created in the hypervariable segments of the Fv region of an antibody and screened for proteolytic activity. Representative catalytic antibodies include oxidoreductases, transferases, hydrolases, lyases, isomerases and ligases; these categories include proteases, carbohydrases, lipases, dioxygenases and peroxidases, as well as other enzymes. These and other enzymes can be used for enzymatic conversions in health care, cosmetics, foods, brewing, detergents, environment (e.g., wastewater treatment), agriculture, tanning, textiles, and other chemical processes, such as diagnostic and therapeutic applications, conversions of fats, carbohydrates and protein, degradation of organic pollutants and synthesis of chemicals. For example, therapeutically effective proteases with fibrinolytic activity, or activity against viral structures necessary for infectivity, such as viral coat proteins, could be engineered. Such proteases could be useful anti-thrombotic agents or anti-viral agents against viruses such as AIDS, rhinoviruses, influenza, or hepatitis. Alternatively, in another example, oxygenases (e.g., dioxygenases), a class of enzymes requiring a co-factor for oxidation of aromatic rings and other double bonds, have industrial applications in biopulping processes, conversion of biomass into fuels or other chemicals, conversion of waste water contaminants, bioprocessing of coal, and detoxification of hazardous organic compounds.

The libraries of the invention relate to a single prototype immunoglobulin of interest. The "prototype" immunoglobulin is the immunoglobulin (or Fab fragment, as described above) upon which all subsequent mutations are based.

### Walk-through Mutagenesis

To prepare the libraries of the invention, "walk-through mutagenesis" is performed on the prototype immunoglobulin. Walk-through mutagenesis is described in detail in U.S. Patents 5,830,650 and 5,798,208.

Although walk-through mutagenesis is equally applicable to proteins and polypeptides other than immunoglobulins, it is discussed herein in reference to mutagenesis of immunoglobulins of interest.

In walk-through mutagenesis, a set (library) of immunoglobulins is generated in which a single predetermined amino acid is incorporated at least once into each position of a defined region (or several defined regions) of interest in the immunoglobulin (i.e., into one or more hypervariable loops (CDRs) of the immunoglobulins). The resultant immunoglobulins (referred to herein as "mutated immunoglobulins") differ from the prototype immunoglobulin, in that they have the single predetermined amino acid incorporated into one or more positions within one or more CDRs of the immunoglobulin, in lieu of the "native" or "wild-type" amino acid which was present at the same position or positions in the prototype immunoglobulin. The set of mutated immunoglobulins includes individual mutated immunoglobulins for each position of the defined region of interest; thus, for each position in the defined region of interest (e.g., the CDR) each mutated immunoglobulin has either an amino acid found in the prototype immunoglobulin, or the predetermined amino acid, and the mixture of all mutated immunoglobulins contains all possible variants.

The predetermined amino acid can be a naturally occurring amino acid. The twenty naturally occurring amino acids differ only with respect to their side chain. Each side chain is responsible for chemical properties that make each amino acid unique (see, e.g., Principles of Protein Structure, 1988, by G. E. Schulz and R. M. Schimer, Springer-Verlag). Typical polar and neutral side chains are those of Cys, Ser, Thr, Asn, Gln and Tyr. Gly is also considered to be a borderline member of this group. Ser and Thr play an important role in forming hydrogen-bonds. Thr has an additional asymmetry at the beta carbon, therefore only one of the stereoisomers is used. The acid amide Gln and Asn can also form hydrogen bonds, the amido groups functioning as hydrogen donors and the carbonyl groups functioning as acceptors. Gln has one more CH2 group than Asn, which renders the polar group more flexible and reduces its interaction with the main chain. Tyr has a very polar hydroxyl group (phenolic OH) that can dissociate at high pH values. Tyr behaves somewhat like a charged side chain; its hydrogen bonds are rather strong.

Neutral polar acids are found at the surface as well as inside protein molecules. As internal residues, they usually form hydrogen bonds with each other or with the polypeptide backbone. Cys can form disulfide bridges. Histidine (His) has a heterocyclic aromatic side chain with a pK value of 6.0. In the physiological pH range, its imidazole ring can be either uncharged or charged, after taking up a hydrogen ion from the solution. Since these two states are readily available, His is quite helpful in catalyzing chemical reactions, and is found in the active centers of many enzymes.

Asp and Glu are negatively charged at physiological pH. Because of their short side chain, the carboxyl group of Asp is rather rigid with respect to the main chain; this may explain why the carboxyl group in many catalytic sites is provided by Asp rather than by Glu. Charged acids are generally found at the surface of a protein.

Lys and Arg are frequently found at the surface. They have long and flexible side chains. Wobbling in the surrounding solution, they increase the solubility of the protein globule. In several cases, Lys and Arg take part in forming internal salt bridges or they help in catalysis. Because of their exposure at the surface of the proteins, Lys is a residue more frequently attacked by enzymes which either modify the side chain or cleave the peptide chain at the carbonyl end of Lys residues.

Using walk-through mutagenesis, a set of nucleic acids (e.g., CDNA) encoding each mutated immunoglobulin can be prepared. In one embodiment, a nucleic acid encoding a mutated immunoglobulin can be prepared by joining together nucleotide sequences encoding regions of the immunoglobulin that are not targeted by walk-through mutagenesis (e.g., constant regions), with nucleotide sequences encoding regions of the immunoglobulin that are targeted by the walk-through mutagenesis (e.g., CDRs). For example, in one embodiment, a nucleic acid encoding a mutated immunoglobulin can be prepared by joining together nucleotide sequences encoding the constant regions of the immunoglobulin, with nucleotide sequences encoding the variable regions. Alternatively, in another example, a nucleic acid encoding a mutated immunoglobulin can be prepared by joining together nucleotide sequences encoding the constant regions, nucleotide sequences encoding portions of the variable regions which are not altered during the walk-through mutagenesis (e.g., oligonucleotides which are outside the CDRs), and the nucleotide sequences encoding the CDRs (e.g., oligonucleotides which are subjected to incorporation of nucleotides that encode the predetermined amino acid). In yet another embodiment, nucleotide sequences encoding the CDRs (e.g., oligonucleotides which are subjected to incorporation of nucleotides that encode the predetermined amino acid) can be individually inserted into a nucleic acid encoding the prototype immunoglobulin, in place of the nucleotide sequence encoding the amino acid sequence of the hypervariable loop (CDR). If desired, the nucleotide sequences encoding the CDRs can be made to contain flanking recognition sites for restriction enzymes (see, e.g., U.S. Pat. No. 4,888,286), or naturally-occurring restriction enzyme recognition sites can be used. The mixture of oligonucleotides can be introduced subsequently by cloning them into an appropriate position using the restriction enzyme sites.

For example, a mixture of oligonucleotides can be prepared, in which each oligonucleotide encodes either a CDR of the prototype immunoglobulin (or a portion of a CDR of the prototype immunoglobulin),or a nucleotide(s) that encode the predetermined amino acid in lieu of one or more native amino acids in the CDR. The mixture of oligonucleotides can be produced in a single synthesis by incorporating, at each position within the oligonucleotide, either a nucleotide required for synthesis of the amino acid present in the prototype immunoglobulin or (in lieu of that nucleotide) a single appropriate nucleotide required for a codon of the predetermined amino acid. The synthesis of the mixture of oligonucleotides can be performed using an automated DNA synthesizer programmed to deliver either one nucleotide to the reaction chamber (e.g., the nucleotide present in the prototype immunoglobulin at that position in the nucleic acid encoding the CDR), or a different nucleotide to the reaction chamber (e.g., a nucleotide not present in the prototype immunoglobulin at that position), or a mixture of the two nucleotides in order to generate an oligonucleotide mixture comprising not only oligonucleotides that encode the CDR of the prototype immunoglobulin, but also oligonucleotides that encode the CDR of a mutated immunoglobulin.

For example, a total of 10 reagent vessels, four of which containing the individual bases and the remaining 6 containing all of the possible two base mixtures among the 4 bases, can be employed to synthesize any mixture of oligonucleotides for the walk-through mutagenesis process. For example, the DNA synthesizer can be designed to contain the following ten chambers:

**Table 1: Synthons for Automated DNA Synthesis**

| Chamber | Synthon |
|---|---|
| 1 | A |
| 2 | T |
| 3 | C |
| 4 | G |
| 5 | (A+T) |
| 6 | (A+C) |
| 7 | (A+G) |
| 8 | (T+C) |
| 9 | (T+G) |
| 10 | (C+G) |

With this arrangement, any nucleotide can be replaced by either one of a combination of two nucleotides at any position of the sequence. Alternatively, if mixing of individual bases in the lines of the oligonucleotide synthesizer is possible, the machine can be programmed to draw from two or more reservoirs of pure bases to generate the desired proportion of nucleotides.

In one embodiment, the two nucleotides (i.e., the wild-type nucleotide and a non-wild-type nucleotide) are used in approximately equal concentrations for the reaction so that there is an equal chance of incorporating either one into the sequence at the position. Alternatively, the ratio of the concentrations of the two nucleotides can be altered to increase the likelihood that one or the other will be incorporated into the oligonucleotide. Alterations in the ratio of concentrations (referred to herein as "doping") is discussed in greater detail in US 20040033569 A.

In another embodiment, solid phase beta-cyanoethyl phosphoramidite chemistry can be used in lieu of automated DNA synthesis for the generation of the oligonucleotides described above (see, e.g., U.S. Patent 4,725,677).

Alternatively, in another embodiment, ribosome expression can be used (see, e.g., Hanes and Pluckthun, "In vitro selection and evolution of functional proteins by using ribosome display", Proc. Natl. Acad Sci. USA, 94:4937-4942 (1997); Roberts and Szostak, "RNA-peptide fusions for the in vitro selection of peptides and proteins", Proc. Natl. Acad Sci. USA, 94: 12297-12302 (1997); Hanes et al., "Picomolar affinity antibodies from a fully synthetic naive library elected and evolved by ribosome display", Nature Biochemistry 18:1287-1292 (2000)).

A library containing nucleic acids encoding mutated immunoglobulins can then be prepared from such oligonucleotides, as described above, and a library containing mutated immunoglobulins can then be generated from the nucleic acids, using standard techniques. For example, the nucleic acids encoding the mutated immunoglobulins can be introduced into a host cell for expression (see, e.g., Huse, W. D. et a/., Science 246:1275 (1989); Viera, J. et al., Meth. Enzymol. 153: 3 (1987)). The nucleic acids can be expressed, for example, in an *E. coli* expression system (see, e.g., Pluckthun, A. and Skerra, A., Meth. Enzymol. 178:476-515 (1989); Skerra, A. et al., Biotechnology 9:23-278 (1991)). They can be expressed for secretion in the medium and/or in the cytoplasm of bacteria (see, e.g., Better, M. and Horwitz, A., Meth. Enzymol. 178:476 (1989)); alternatively, they can be expressed in other organisms such as yeast or mammalian cells (e.g., myeloma or hybridoma cells).

One of ordinary skill in the art will understand that numerous expression methods can be employed to produce libraries described herein. By fusing the gene (library) to additional genetic elements, such as promoters, terminators, and other suitable sequences that facilitate transcription and translation, expression in vitro (ribosome display) can be achieved as described by Pluckthun et al.(Pluckthun, A. and Skerra, A., Meth. Enzymol. 178:476-515 (1989)). Similarly, Phage display, bacterial expression, baculovirus-infected insect cells, fungi (yeast), plant and mammalian cell expression can be obtained as described (Antibody Engineering. R. Konterman, S.Dubel (Eds.). Springer Lab manual. Spriger-Verlag. Berlin, Heidelberg (2001), Chapter 1, "Recombinant Antibodies by S. Dubel and R. E. Konterman. Pp. 4-16). Libraries of scFV can also be fused to other genes to produce chimaeric proteins with binding moieties (Fv) and other functions, such as catalytic, cytotoxic, etc. (Antibody Engineering. R. KONTERMAN, S.Dubel (Eds.). Springer Lab manual. Spriger-Verlag. Berlin, Heidelberg (2001), Chapter 41. Stabilization Strategies and Application of recombinant Fvs and Fv Fusion proteins. By U. Brinkmann, pp. 593-615).

### Preparation of the Universal Library

To generate a library for the immunoglobulin of interest, walk-through mutagenesis using a single predetermined amino acid is performed for the prototype immunoglobulin, producing individual nucleic acid libraries comprising nucleotides encoding mutated immunoglobulins (and also nucleotides encoding prototype immunoglobulin). The nucleic acid libraries can be translated to form amino acid libraries comprising mutated immunoglobulin proteins (referred to herein as "single predetermined amino acid libraries"). Each single predetermined amino acid library contains 64 subset libraries, in which the predetermined amino acid is "walked through" each hypervariable loop (CDR) of the immunoglobulin of interest (that is, the three hypervariable loops in the variable region of the heavy chain (VH1, VH2 and VH3), and in the three hypervariable loops in the variable region of the light chain (VL1, VL2 and VL3)). The resultant immunoglobulins include mutated immunoglobulins having the predetermined amino acid at one or more positions in each CDR, and collectively having the predetermined amino acid at each position in each CDR. The single predetermined amino acid is "walked through" each of the six hypervariable loops (CDR) individually; and then through each of the possible combinatorial variations of the CDRs (pairs, triad, tetrads, etc.). The possible combinatorial variations are set forth in Table 2:

**Table 2: Subset Libraries for each Single Predetermined Amino Acid Library**

| Subset Library | Number of Hypervariable Regions (CDRs) | Number of Libraries |
|---|---|---|
| A | 1 | 6 (VH1, VH2, VH3, VL1, VL2 or VL3) |
| B | 2 | 15 (all possible combinations of 2) |
| C | 3 | 20 (all possible combinations of 3) |
| D | 4 | 15 (all possible combinations of 4) |
| E | 5 | 6 (all possible combinations of 5) |
| F | 6 | 1 (VH1, VH2, VH3, VL1, VL2 and VL3) |

Total: 63 subset libraries. A 64^{th} subset library includes the prototype immunoglobulin.

To prepare a "universal" library for the prototype immunoglobulin of interest, walk-through mutagenesis using a single predetermined amino acid is performed for the prototype immunoglobulin, for each of the twenty natural amino acids, producing 20 individual "single predetermined amino acid libraries," as described above. These 20 individual "single predetermined amino acid libraries" collectively form a universal library for the immunoglobulin of interest.

Thus, in total, the universal library for an immunoglobulin of interest contains 20 (single predetermined amino acid) libraries which each include 64 subset libraries, for a total of 1208 libraries.

### Library Uses

Libraries as described herein contain mutated immunoglobulins which have been generated in a manner that allows systematic and thorough analysis of the binding regions of the prototype immunoglobulin, and particularly, of the influence of a particular preselected amino acid on the binding regions. The libraries avoid problems relating to control or prediction of the nature of a mutation associated with random mutagenesis; allow generation of specific information on the particular mutations that allow altered interaction of the immunoglobulin of interest with its antigen, including multiple interactions by amino acids in the varying complementarity-determining regions.

The libraries can be screened by appropriate means for particular immunoglobulins having specific characteristics. For example, catalytic activity can be ascertained by suitable assays for substrate conversion and binding activity can be evaluated by standard immunoassay and/or affinity chromatography. Assays for these activities can be designed in which a cell requires the desired activity for growth. For example, in screening for immunoglobulins that have a particular activity, such as the ability to degrade toxic compounds, the incorporation of lethal levels of the toxic compound into nutrient plates would permit the growth only of cells expressing an activity which degrades the toxic compound (Wasserfallen, A., Rekik, M., and Harayama, S., Biotechnology 9: 296-298 (1991)). Libraries can also be screened for other activities, such as for an ability to target or destroy pathogens. Assays for these activities can be designed in which the pathogen of interest is exposed to the antibody, and antibodies demonstrating the desired property (e.g., killing of the pathogen) can be selected.

Information relative to the effect of the specific amino acid included in the CDR regions, either as single or as multiple amino acid substitutions, provides unique information on the specific effect of a given amino acid as related to affinity and specificity between the antibody and the antigen (antibody maturation or optimization).

In addition, the presence or the enrichment of specific amino acids in the binding regions of an antibody (immunoglobulin) molecule provides new sequences (amino acid domains) capable of interacting with a variety of new antigen for antibody discovery.

The following Exemplification is offered for the purpose of illustrating the present invention and are not to be construed to limit the scope of this invention.

### EXEMPLIFICATION

### A. Material and Methods

The follow example illustrates the synthesis of gene libraries by the walk-through mutagenesis (WTM) including the design and synthesis of universal amino acid libraries. The construction of these libraries was based upon the amino acid sequence of a human anti HIV GP120 monoclonal antibody, specifically limited to its Fv (VL and VH) regions, designed as single chain (scFV). The amino acid sequence of the VL and VH regions of GP-120 monoclonal antibody was obtained by a human sequence published in the literature (Antibody Engineering. R. KONTERMAN, S.Dubel (Eds.). Springer Lab manual. Spriger-Verlag. Berlin, Heidelberg (2001), Chapter 1, "Recombinant Antibodies" by S. Dubel and R. E. Konterman. pp. 4-16.).

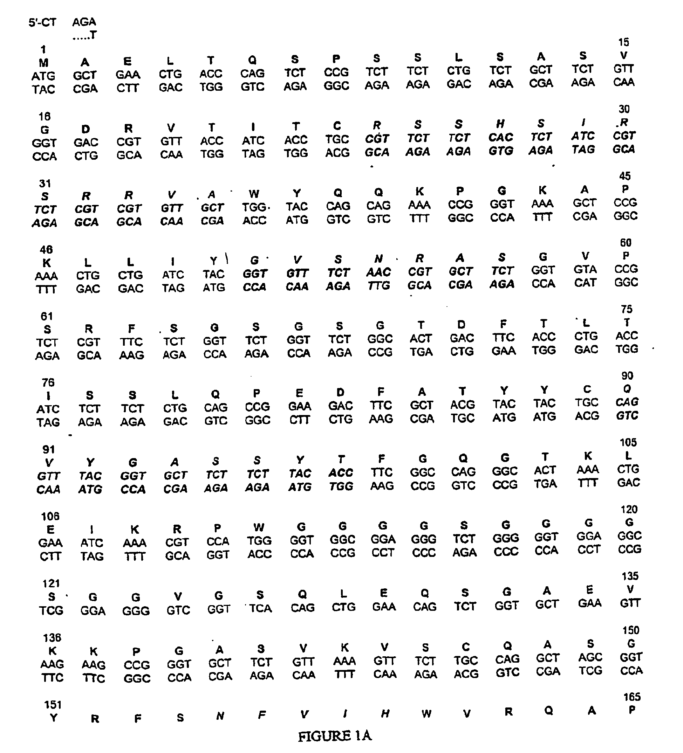

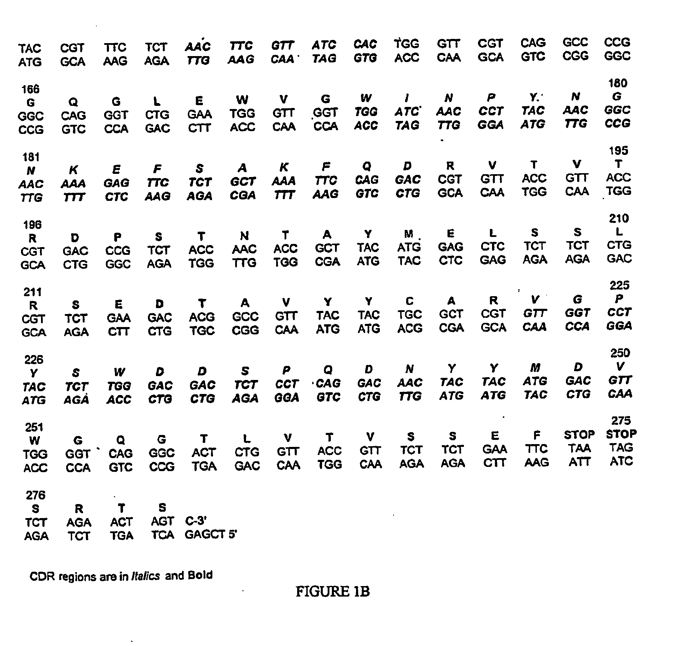

FIG.1A-1B show the complete sequence (amino acids and DNA) of the GP-120 Fv organized as single chain (scFv). The complete DNA sequence was obtained by artificially connecting the C-terminus of VL gene to the N-terminus of VH gene with a DNA sequence coding for a synthetic peptide (G4S)3 as reported previously (Huston, JS, Levinson D, Mudgett-Hunter M, Tai MS, Novotny J, Margulis MN, Ridge RJ, Bruccoleri RE, Haber EC, Crea R, and Opperman H, Protein engineering of antibody binding site: recovery of specific activity in an anti-digioxin single-chain Fv analogue produced in E.Coli. Proc Nat Acad Sci USA 85, 5879-5883, 1988; Bird RE, Hardman KD, Jacobson JW, Johnson S, Kaufman BM, Lee SM, Pope SH, Riordan GS and Witlow M, Single-chain antigen binding proteins. Science 242, 423-426, 1988.). The VL and VH amino acid sequences are numbered according to Kabat *et al.* (Kabat EA, Wu TT, Reid-Miller M, Perry HM, Gottesman KS, Foeller C, (1991) Sequences of proteins of Immunological Interest. 5th Edition. US Department Of Health and Human Services, Public Service, NIH.). The CDR regions (L1,L2,L3 and H1,H2, H3) are shown in bold.

The DNA sequence for VL and VH were redesigned to make use of the most frequent a.a.codons in *E.coli.* Furthermore, several restriction enzyme sites were included in the sequence to facilitate R.E. analysis. 5-Sticky ends (Xba1, HindIII, and Sal I) and two codons for termination (TAA, TAG) were also incorporated in the scFV gene sequence to facilitate cloning, sequencing and expression in readily available commercial plasmids.

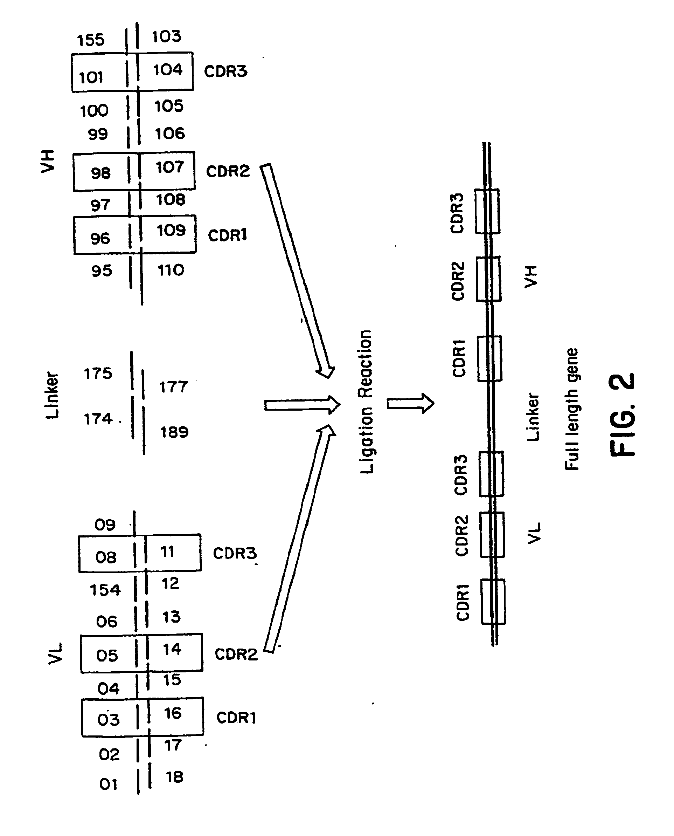

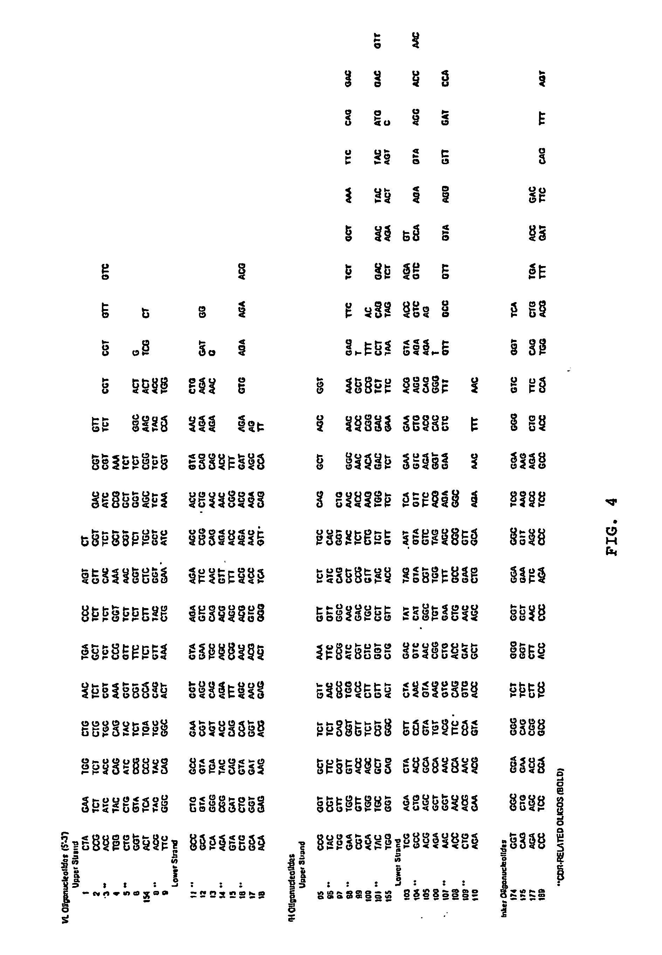

The overall assembly scheme for the GP-120 scFV gene was obtained from synthetic oligonucleotides, as schematically shown in FIG.2. The complete assembly was designed to include the fusion (ligation) of independently assembled VL and VH genes. This latter was achieved by enzymatic ligation (T4-ligase) of appropriately overlapping synthetic oligonucleotides as shown in FIG. 4. Upon isolation of the VL and VH genes by preparative gel electrophoresis and further ligation by the aid of synthetic oligonucleotides (#174,175,177 and 189) coding for the linker (G4S)3 in the presence of Ligase gave the scFv construct.

Oligonucleotide synthesis was performed on an Eppendorf D-300 synthesizer following the procedure provided by the vendor. Each oligonucleotide was purified by gel electrophoresis, desalted by quick passage through a Sephadex based mini-column and stored individually at a concentration equal to 5 O.D. u/ml.

Enzymatic ligation of VL and VH genes was performed under standard conditions (Maniatis et al.) where all the VL and VH oligonucleotides, with the exception of the 5'-end of upper and lower strands, were first phosphorylated by T-4 Kinase, and used in equimolar concentration for gene assembly in the presence of T4-ligase and ATP. The final assembly of scFV was obtained by the ligation of an equimolar amount of VL and VH in the presence of an excess (10x) of the oligo linkers. The final scFV was first amplified by the use of DNA-polymerase in the presence of NTP and the fragments # 201 and #103, and then purified by preparative gel electrophoresis.

The correctness of the scFV gene was confirmed by DNA sequencing analysis, using an Applied Biosystems automatic DNA sequencer, following standard conditions provided by the vendor.

To generate GP-120 scFv gene libraries containing selected amino acids in some of the CDR regions of the scFV protein, synthetic oligonucleotide pools corresponding to the target CDR regions were designed and synthesized following the rules dictated by the walk through mutagenesis process (as described herein; see also U.S. Patents 5,830,650 and 5,798.208) using an Eppendorf D300 synthesizer.

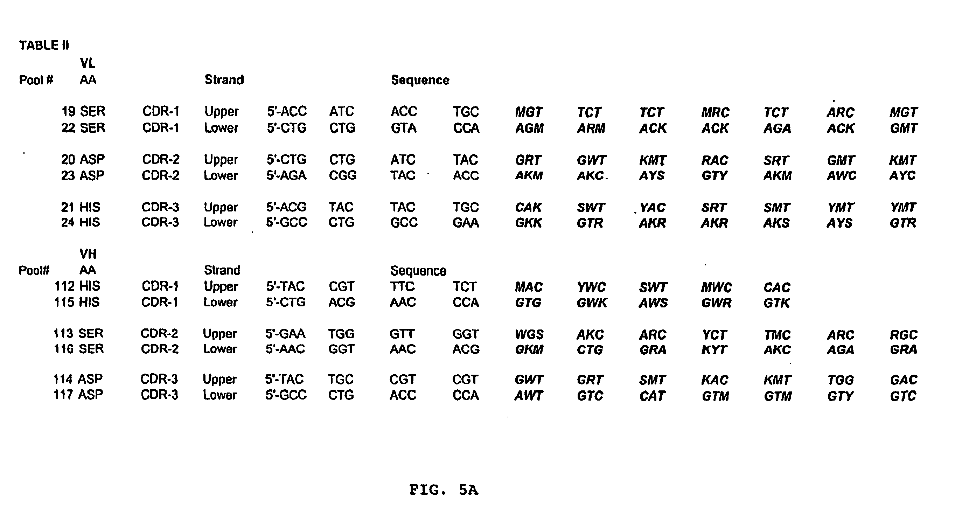

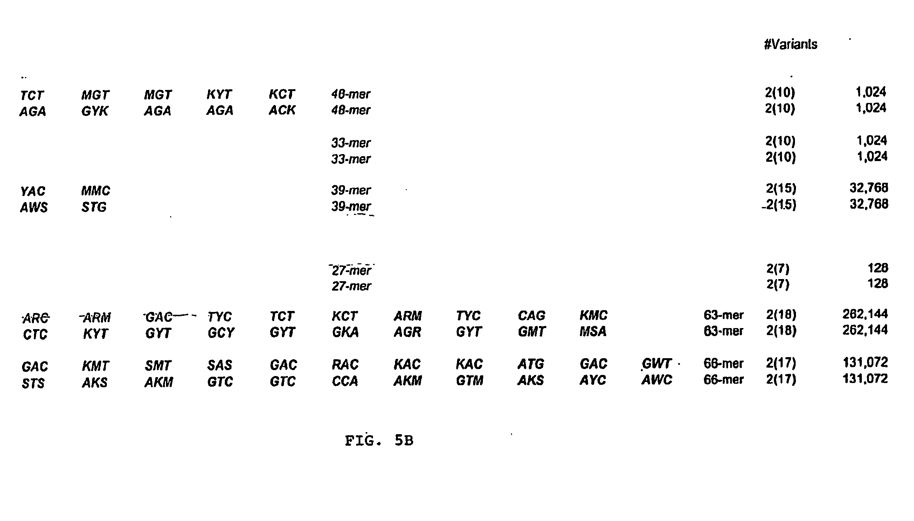

FIG. 5 illustrates examples of oligonucleotides pools designed to introduce three (3) targeted amino acid, SER, HIS and ASP, in individual CDRs of the Fv, in a number of possible combinations. The oligonucleotide pools were produced by the mixing of equal amount of activated nucleoside phosphoramidates during the chemical synthesis. The pool sequences in FIG. 5 are given using the IUPAC nomenclature of mixed bases (show in bold capital letters, R= A or G, Y= C or T, M = A or C, K = G or T, S = C or G, W = A or T; H = A or C or T, B = C or G or T, V = A or C or G, D= A or G or T.

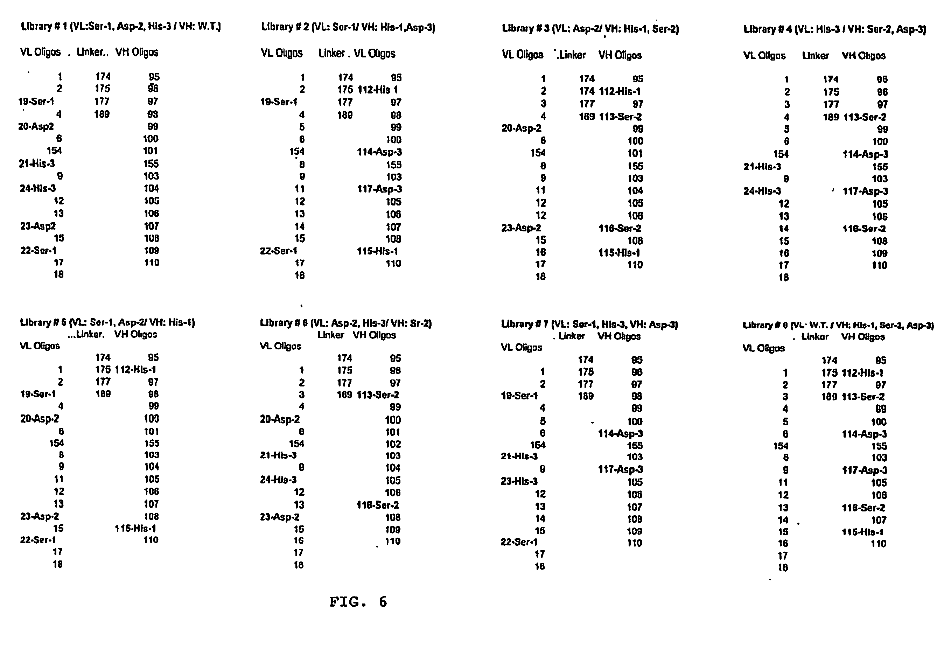

FIG. 6 illustrates the strategy adopted for VL and VH gene assembly in order to generate libraries of GP-120 scFV in which three (3) CDR regions out of the six, were contemporaneously mutagenized to produce the presence of selected individual amino acids (Ser, His and Asp) in a number (8) of different combinations (L1 to L8).

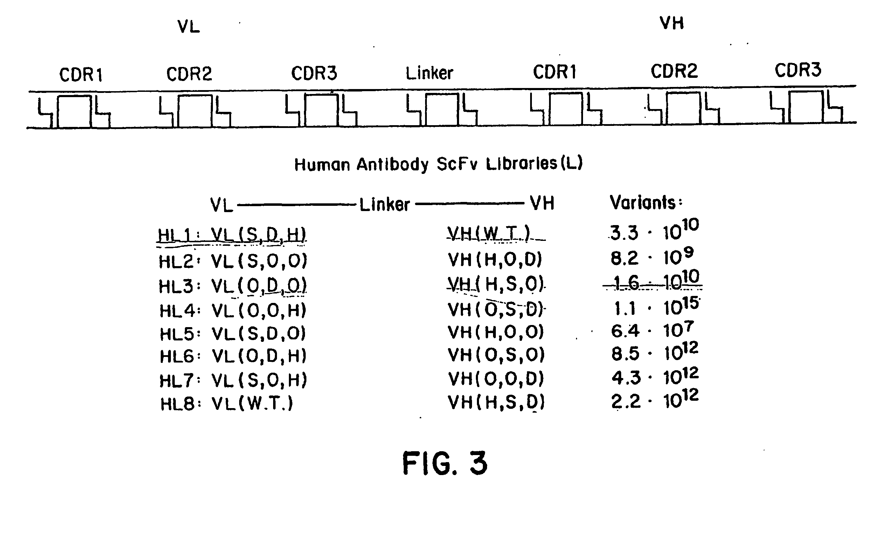

Fig.3 summarizes the resulting scFV gene libraries obtained by the above strategy and the number of gene variants produced for each individual library.

Individual scFV libraries can be cloned in suitable sequencing and/or expression plasmids. Thus, sequencing analysis and gene expression can be obtained accordingly. In this example, a pFLAG plasmid was employed as sequencing plasmid, while the plasmid pCANTAB 5E was used to obtain expression of the scFV gene libraries in E.coli (periplasmic space).

### B. Design and synthesis of universal amino acid libraries

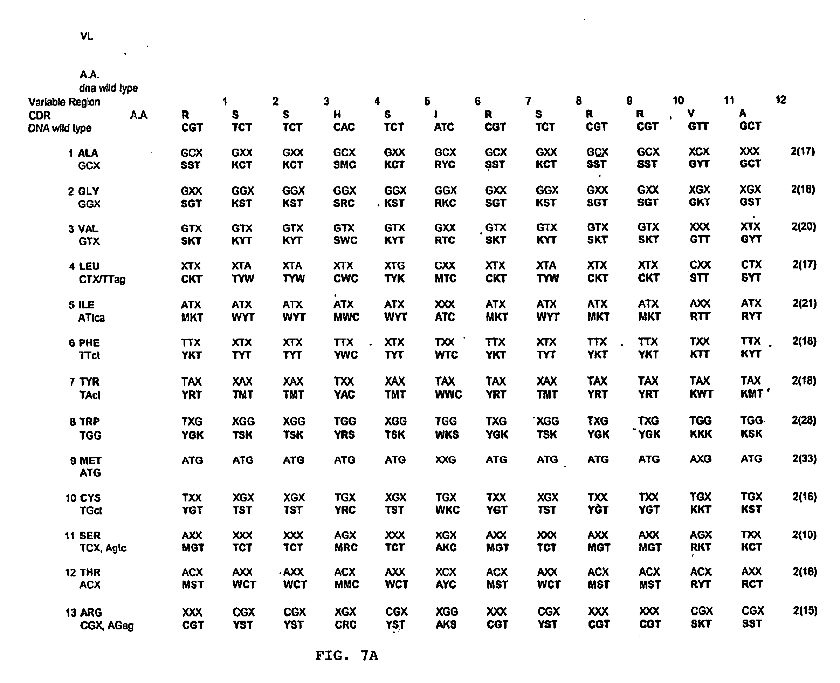

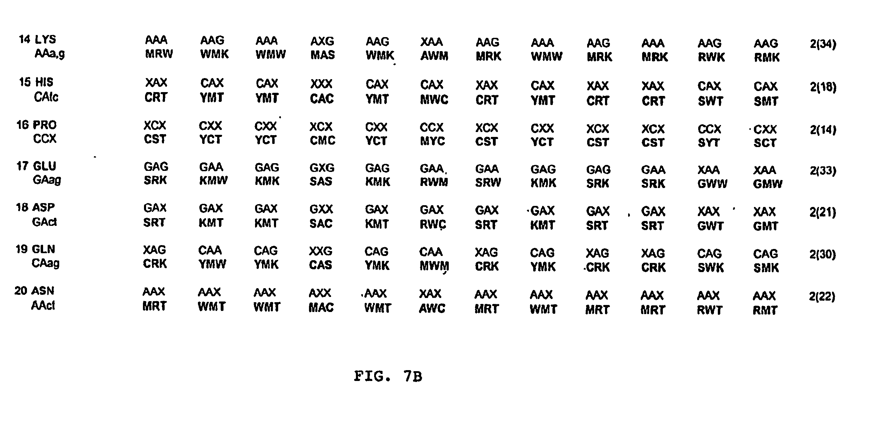

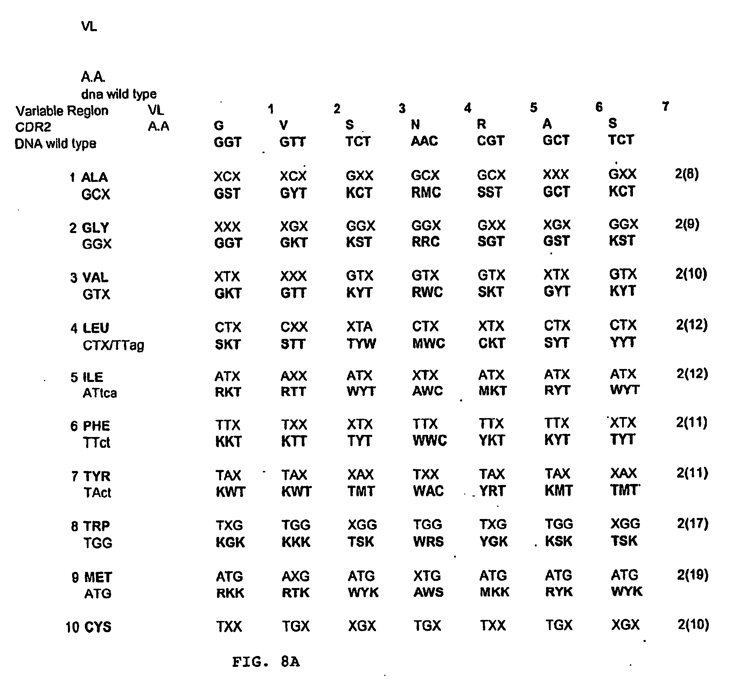

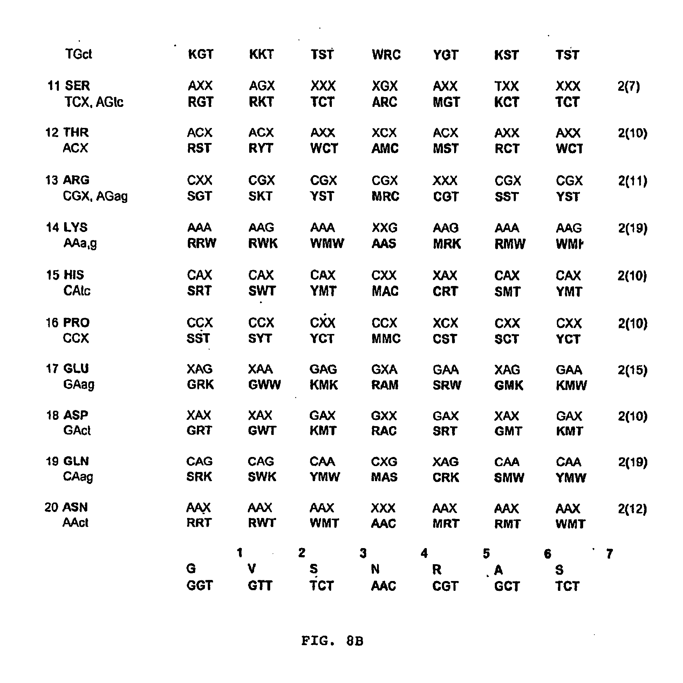

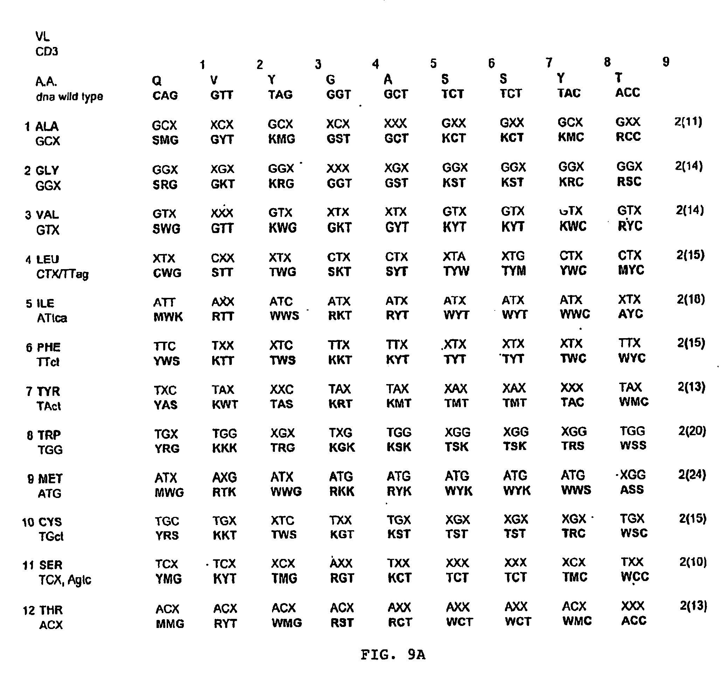

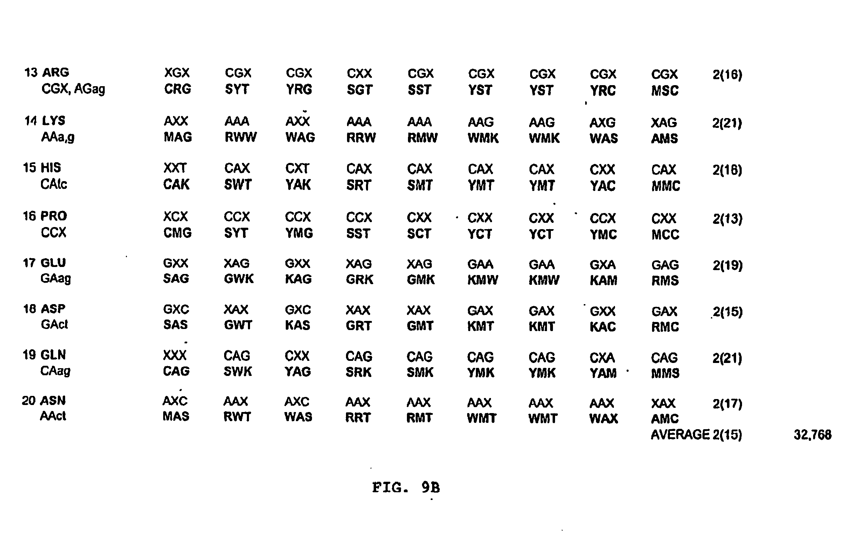

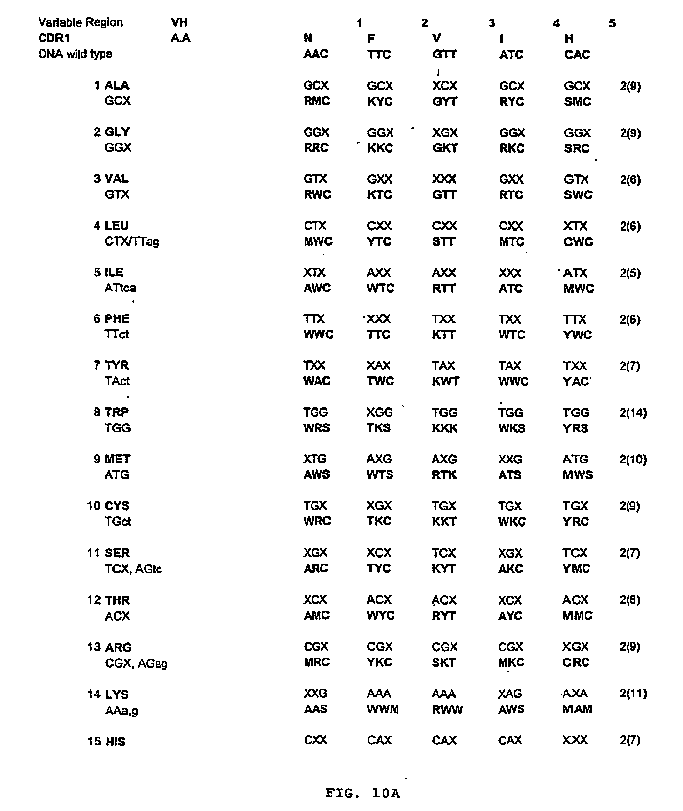

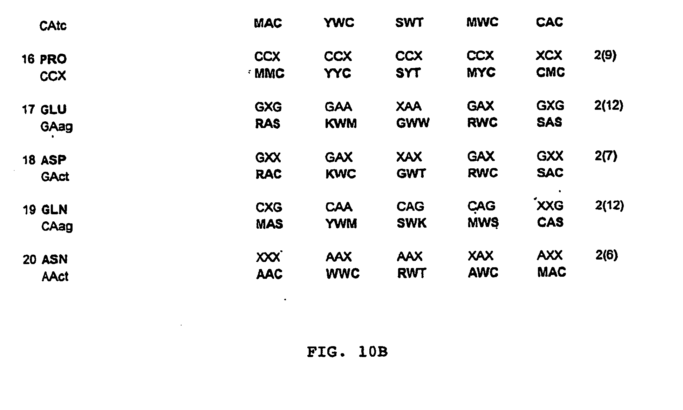

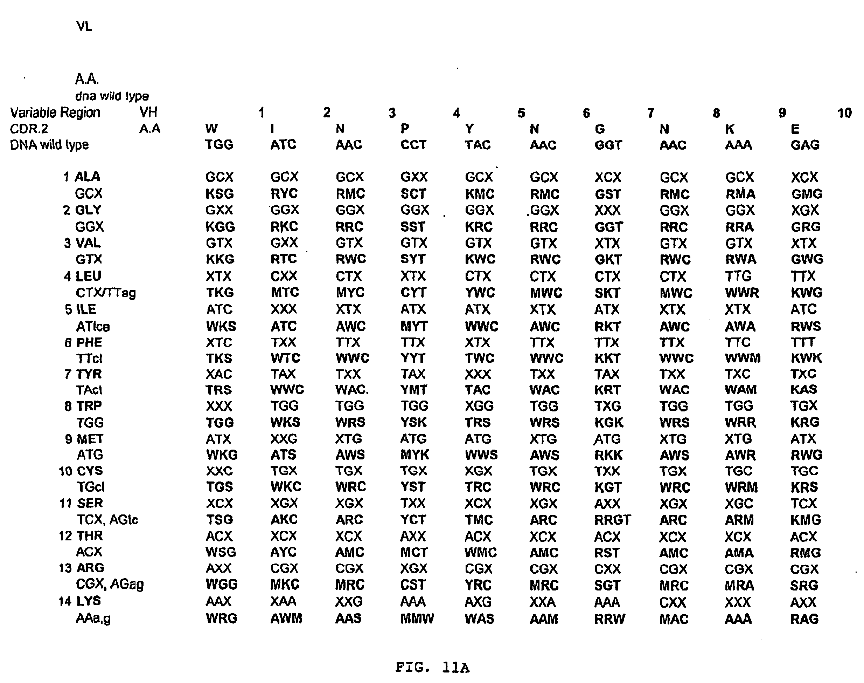

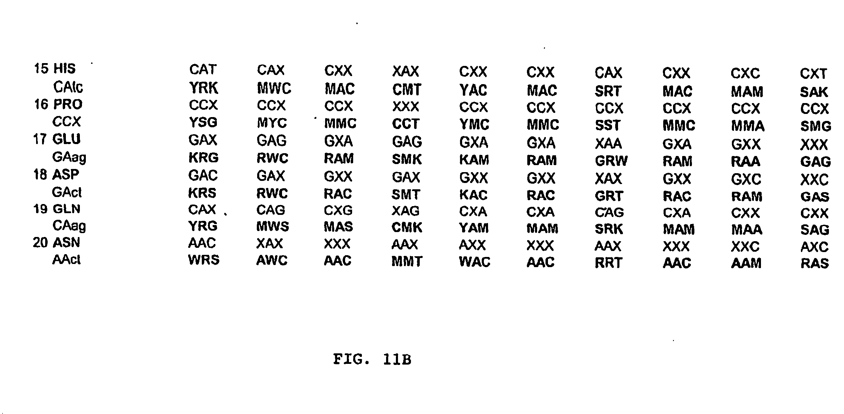

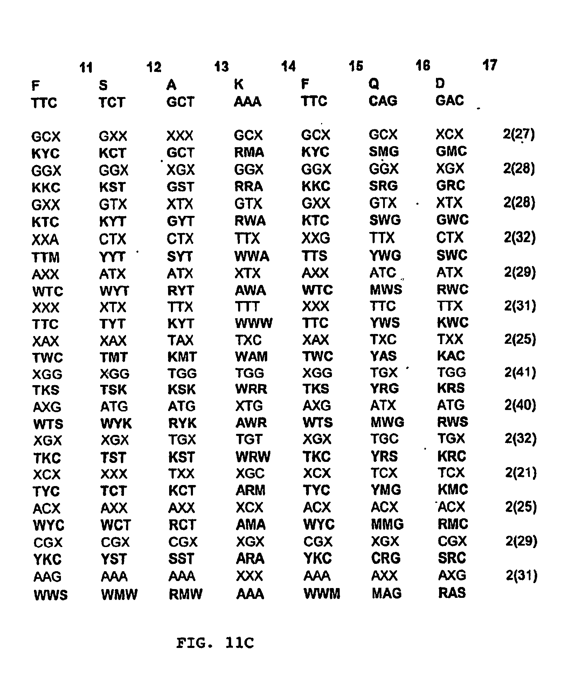

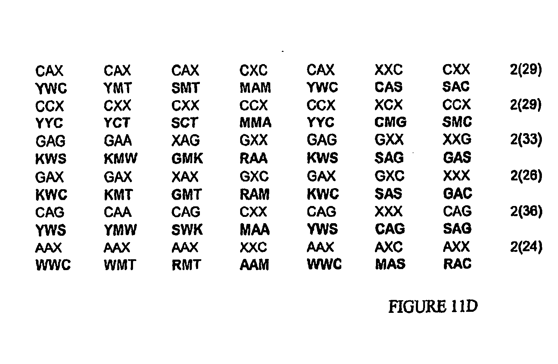

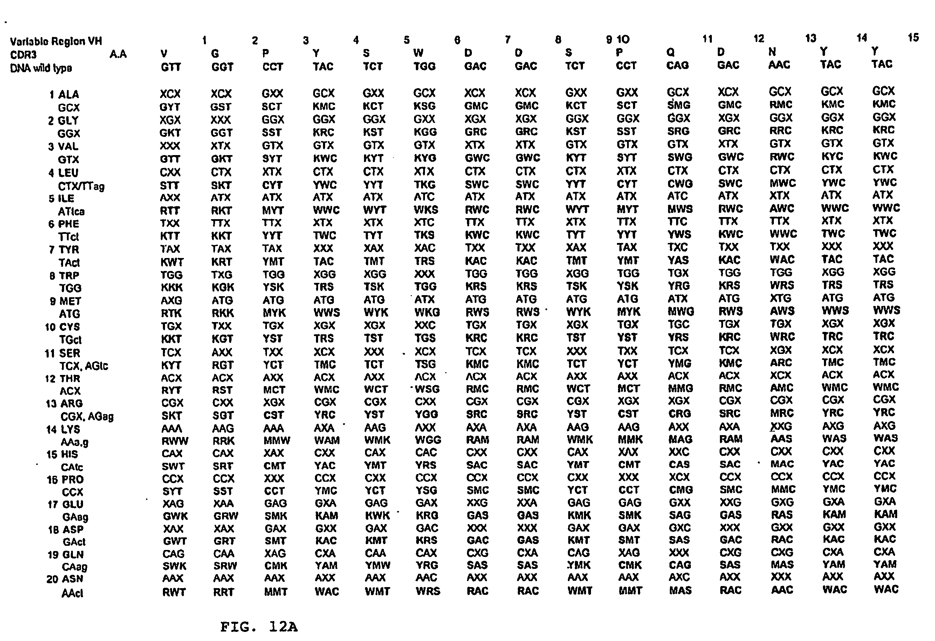

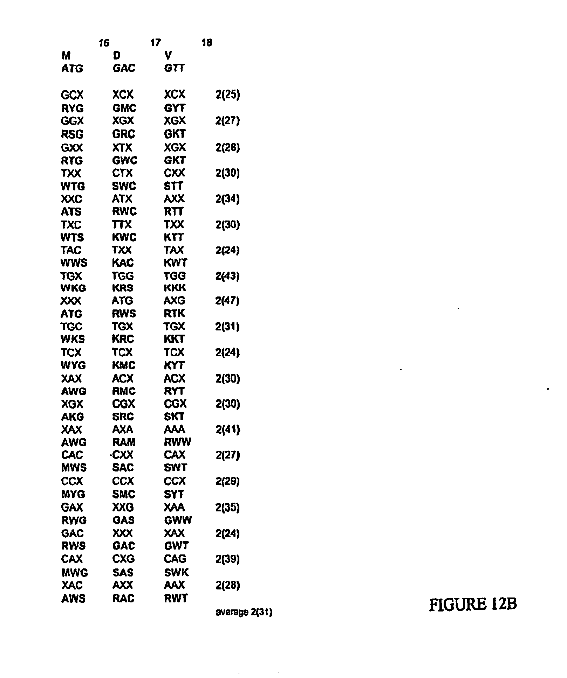

Using the methods described above, 20 individual oligonucleotide pools, each corresponding to one of the 20 natural amino acids, can be designed for each of the six CDRs, as illustrated in FIG. 7-12. From the compilation of these oligo pools, the six (6) pools corresponding to each selected amino acid (any of the 20 natural amino acids) can be used in any possible combinatorial arrangement to mutagenize the corresponding CDR regions of the scFV gene.

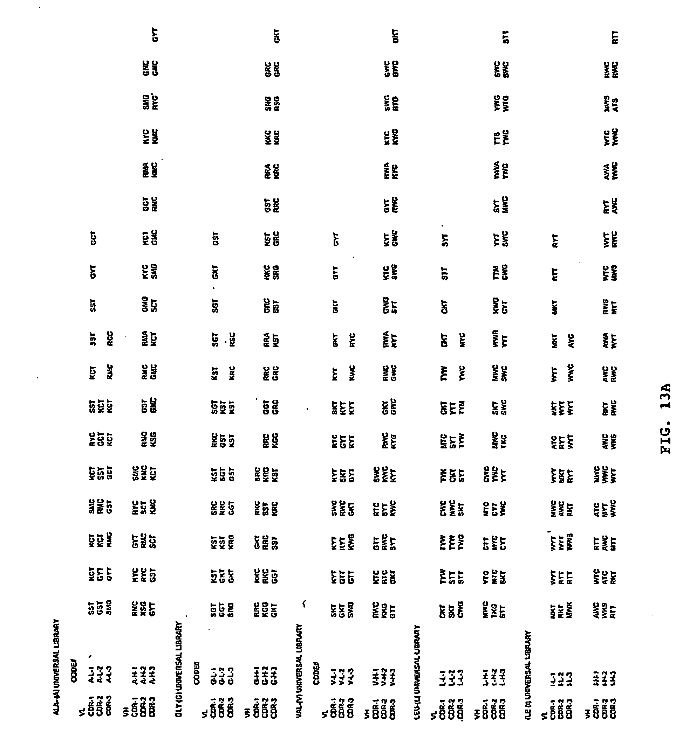

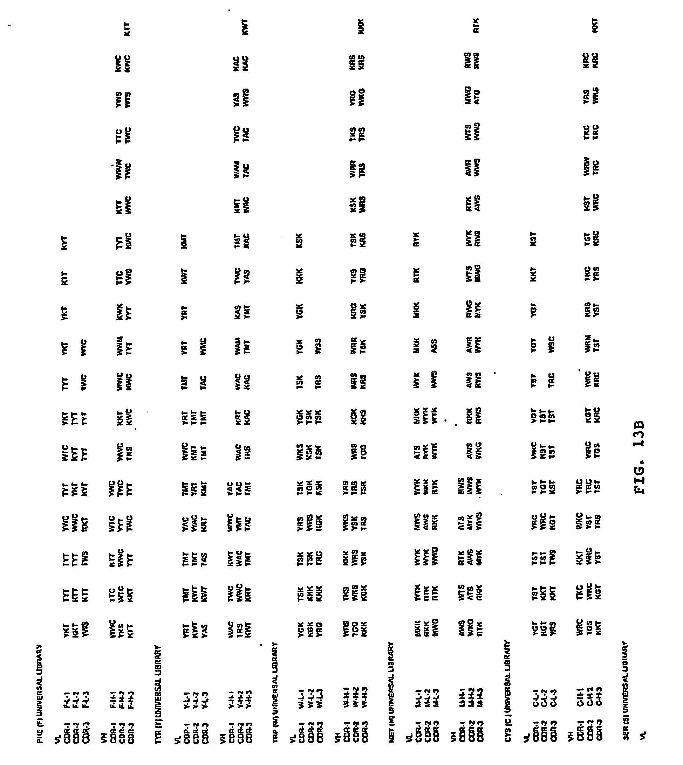

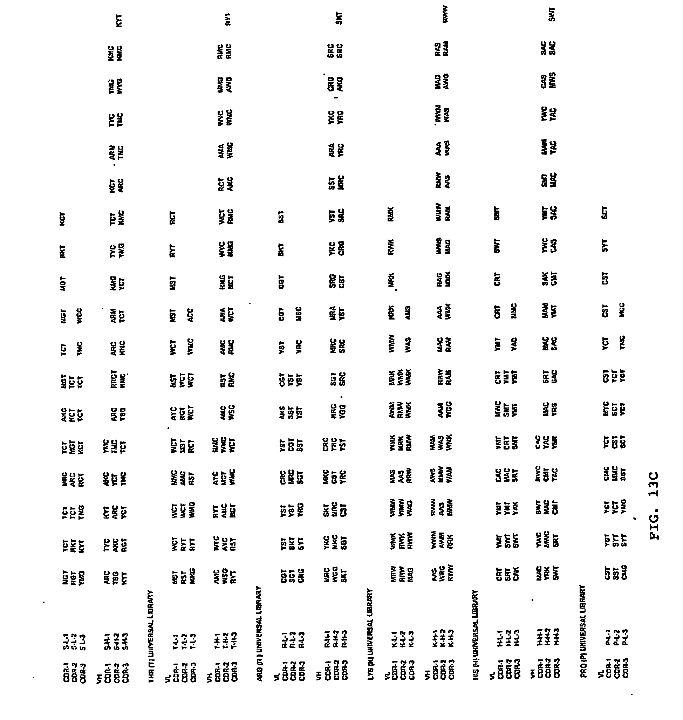

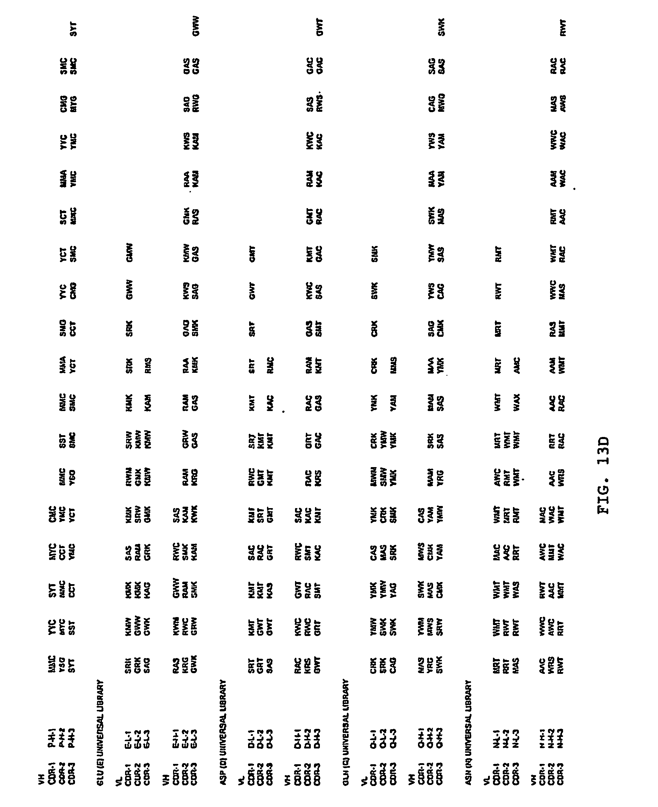

FIG. 13 shows the grouping of the CDR pools for individual amino acids. The six pools can be used in any combinatorial formula, from single CDR replacement (six individual libraries) to total saturation (ALL six CDR regions mutagenized) and any combination in between, as described above.

Each and any of the resulting libraries (63 in total+ one wild type sequence) will contain only pool(s) of oligonucleotides designed to provide a selected amino acid, which therefore becomes systematically distributed in the six CDR regions of the scFv gene, as described above. As result of this synthetic scheme, gene libraries containing in prevalence one selected amino acid, distributed throughout the six CDR regions in any combinatorial way, will be obtained as individual entities and separated libraries.

While this invention has been particularly shown and described with references to preferred embodiments thereof, it will be understood by those skilled in the art that various changes in form and details may be made therein without departing from the spirit and scope of the invention as defined by the appended claims.

## Claims

1. A single predetermined amino acid library comprising immunoglobulins of interest with six complementarity-determining regions, the library including subset libraries comprising:
a) one subset library comprising a prototype immunoglobulin of interest, and upon which all subsequent mutations are based; and
b) six subset libraries, each comprising mutated immunoglobulins in which a single predetermined amino acid has been substituted in one or more positions in one of the six complementarity-determining regions of the prototype immunoglobulin, with one subset library for each of the six complementarity-determining regions; and
c) 15 subset libraries, each comprising mutated immunoglobulins in which the single predetermined amino acid has been substituted in one or more positions in two of the six complementarity-determining regions of the prototype immunoglobulin with one subset library for each of the possible combinations of two of the six complementarity-determining regions; and
d) 20 subset libraries, each comprising mutated immunoglobulins in which the single predetermined amino acid has been substituted in one or more positions in three of the six complementarity-determining regions of the prototype immunoglobulin with one subset library for each of the possible combinations of three of the six complementarity-determining regions; and
e) 15 subset libraries, each comprising mutated immunoglobulins in which the single predetermined amino acid has been substituted in one or more positions in four of the six complementarity-determining regions of the prototype immunoglobulin, with one subset library for each of the possible combinations of four of the six complementarity-determining regions; and
f) 6 subset libraries, each comprising mutated immunoglobulins in which the single predetermined amino acid has been substituted in one or more positions in five of the six complementarity-determining regions of the prototype immunoglobulin, with one subset library for each of the possible combinations of five of the six complementarity-determining regions; and
g) one subset library comprising mutated immunoglobulins in which the single predetermined amino acid has been substituted in one or more positions in all of the six complementarity-determining regions of the prototype immunoglobulin;
wherein each subset library that contains mutated immunoglobulins, comprises mutated immunoglobulins in which the single predetermined amino acid is present at least once at every position in the complementarity-determining region into which the single predetermined amino acid has been introduced.

2. A single predetermined amino acid library comprising nucleic acids encoding immunoglobulins of interest with six complementarity-determining regions, the library including subset libraries comprising:
a) one subset library comprising nucleic acids encoding a prototype immunoglobulin of interest upon which all subsequent mutations are based; and
b) six subset libraries, each comprising nucleic acids encoding mutated immunoglobulins in which a single predetermined amino acid has been substituted in one or more positions in one of the six complementarity-determining regions of the prototype immunoglobulin, with one subset library for each of the six complementarity-determining regions; and
c) 15 subset libraries, each comprising nucleic acids encoding mutated immunoglobulins in which the single predetermined amino acid has been substituted in one or more positions in two of the six complementarity-determining regions of the prototype immunoglobulin, with one subset library for each of the possible combinations of two of the six complementarity-determining regions; and
d) 20 subset libraries comprising nucleic acids encoding mutated immunoglobulins in which the single predetermined amino acid has been substituted in one or more positions in three of the six complementarity-determining regions of the prototype immunoglobulin, with one subset library for each of the possible combinations of three of the six complementarity-determining regions; and
e) 15 subset libraries, each comprising nucleic acids encoding mutated immunoglobulins in which the single predetermined amino acid has been substituted in one or more positions in four of the six complementarity-determining regions of the prototype immunoglobulin, with one subset library for each of the possible combinations of four of the six complementarity-determining regions; and
f) 6 subset libraries, each comprising nucleic acids encoding mutated immunoglobulins in which the single predetermined amino acid has been substituted in one or more positions in five of the six complementarity-determining regions of the prototype immunoglobulin, with one subset library for each of the possible combinations of five of the six complementarity-determining regions; and
g) one subset library comprising nucleic acids encoding mutated immunoglobulins in which the single predetermined amino acid has been substituted in one or more positions in all of the six complementarity-determining regions of the prototype immunoglobulin;
wherein each subset library that contains nucleic acids encoding mutated immunoglobulins, comprises nucleic acids encoding mutated immunoglobulins in which the single predetermined amino acid is present at least once at every position in the complementarity-determining region into which the single predetermined amino acid has been introduced.

3. The library of claim 1 or claim 2, wherein the immunoglobulin of interest is a catalytic antibody; IgG; IgM; IgA; IgD; IgE; an Fab fragment of an immunoglobulin; or a single chain immunoglobulin.

4. A universal library comprising:
twenty single predetermined amino acid libraries consisting of one single predetermined amino acid library according to claim 1, 2 or 3 for each of the twenty naturally occurring amino acids.

## Patentansprüche

1. Einzelne vorher bestimmte Aminosäure-Bibliothek, die Immunglobuline von Interesse mit sechs Komplementarität-bestimmenden Regionen umfasst, wobei die Bibliothek Teilbibliotheken enthält, umfassend:
a) eine Teilbibliothek, umfassend ein Prototyp-Immunglobulin von Interesse, auf dem alle nachfolgenden Mutationen basieren; und
b) sechs Teilbibliotheken, jede umfassend mutierte Immunglobuline, in denen eine einzelne vorher bestimmte Aminosäure in einer Position oder mehreren Positionen in einer der sechs Komplementarität-bestimmenden Regionen des Prototyp-Immunglobulins ausgetauscht wurde, mit einer Teilbibliothek für jede der sechs Komplementarität-bestimmenden Regionen; und
c) 15 Teilbibliotheken, jede umfassend mutierte Immunglobuline, in denen die einzelne vorher bestimmte Aminosäure in einer Position oder mehreren Positionen in zwei der sechs Komplementarität-bestimmenden Regionen des Prototyp-Immunglobulins substituiert wurde, mit einer Teilbibliothek für jede der möglichen Kombinationen von zwei der sechs Komplementarität-bestimmenden Regionen; und
d) 20 Teilbibliotheken, jede umfassend mutierte Immunglobuline, in denen die einzelne vorher bestimmte Aminosäure in einer Position oder mehreren Positionen in drei der sechs Komplementarität-bestimmenden Regionen des Prototyp-Immunglobulins ausgetauscht wurde, mit einer Teilbibliothek für jede der möglichen Kombinationen von drei der sechs Komplementarität-bestimmenden Regionen; und
e) 15 Teilbibliotheken, jede umfassend mutierte Immunglobuline, in denen die einzelne vorher bestimmte Aminosäure in einer Position oder mehreren Positionen in vier der sechs Komplementarität-bestimmenden Regionen des Prototyp-Immunglobulins ausgetauscht wurde, mit einer Teilbibliothek für jede der möglichen Kombinationen von vier der sechs Komplementarität-bestimmenden Regionen; und
f) sechs Teilbibliotheken, jede umfassend mutierte Immunglobuline, in denen die einzelne vorher bestimmte Aminosäure in einer Position oder mehreren Positionen in fünf der sechs Komplementarität-bestimmenden Regionen des Prototyp-Immunglobulins ausgetauscht wurde, mit einer Teilbibliothek für jede der möglichen Kombinationen von fünf der sechs Komplementarität-bestimmenden Regionen; und
g) eine Teilbibliothek, umfassend mutierte Immunglobuline, in denen die einzelne vorher bestimmte Aminosäure in einer Position oder mehreren Positionen in allen der sechs Komplementarität-bestimmenden Regionen des Prototyp-Immunglobulins ausgetauscht wurde;
wobei jede Teilbibliothek, die mutierte Immunglobuline enthält, mutierte Immunglobuline umfasst, in denen die einzelne vorher bestimmte Aminosäure wenigstens einmal in jeder Position in der Komplementarität-bestimmenden Region, in die die einzelne vorher bestimmte Aminosäure eingeführt worden ist, vorhanden ist.

2. Einzelne vorher bestimmte Aminosäure-Bibliothek, die Nucleinsäuren umfasst, welche für Immunglobuline von Interesse mit sechs Komplementarität-bestimmenden Regionen codieren, wobei die Bibliothek Teilbibliotheken enthält, umfassend:
a) eine Teilbibliothek, umfassend Nucleinsäuren, die für ein Prototyp-Immunglobulin von Interesse codieren, auf dem alle folgenden Mutationen basieren; und
b) sechs Teilbibliotheken, jede umfassend Nucleinsäuren, die für mutierte Immunglobuline codieren, in denen eine einzelne vorher bestimmte Aminosäure in einer Position oder mehreren Positionen in einer der sechs Komplementarität-bestimmenden Regionen des Prototyp-Immunglobulins ausgetauscht wurde, mit einer Teilbibliothek für jede der sechs Komplementarität-bestimmenden Regionen; und
c) 15 Teilbibliotheken, jede umfassend Nucleinsäuren, die für mutierte Immunglobuline codieren, in denen die einzelne vorher bestimmte Aminosäure in einer Position oder mehreren Positionen in zwei der sechs Komplementarität-bestimmenden Regionen des Prototyp-Immunglobulins ausgetauscht wurde, mit einer Teilbibliothek für jede der möglichen Kombinationen von zwei der sechs Komplementarität-bestimmenden Regionen; und
d) 20 Teilbibliotheken, umfassend Nucleinsäuren, die für mutierte Immunglobuline codieren, in denen die einzelne vorher bestimmte Aminosäure in einer Position oder mehreren Positionen in drei der sechs Komplementarität-bestimmenden Regionen des Prototyp-Immunglobulins ausgetauscht wurde, mit einer Teilbibliothek für jede der möglichen Kombinationen von drei der sechs Komplementarität-bestimmenden Regionen; und
e) 15 Teilbibliotheken, jede umfassend Nucleinsäuren, die für mutierte Immunglobuline codieren, in denen die einzelne vorher bestimmte Aminosäure in einer Position oder mehreren Positionen in vier der sechs Komplementarität-bestimmenden Regionen des Prototyp-Immunglobulins ausgetauscht wurde, mit einer Teilbibliothek für jede der möglichen Kombinationen von vier der sechs Komplementarität-bestimmenden Regionen; und
f) sechs Teilbibliotheken, jede umfassend Nucleinsäuren, die für mutierte Immunglobuline codieren, in denen die einzelne vorher bestimmte Aminosäure in einer Position oder mehreren Positionen in fünf der sechs Komplementarität-bestimmenden Regionen des Prototyp-Immunglobulins ersetzt wurde, mit einer Teilbibliothek für jede der möglichen Kombinationen von fünf der sechs Komplementarität-bestimmenden Regionen; und
g) eine Teilbibliothek, umfassend Nucleinsäuren, die für mutierte Immunglobuline codieren, in denen die einzelne vorher bestimmte Aminosäure in einer Position oder mehreren Positionen in allen der sechs Komplementarität-bestimmenden Regionen des Prototyp-Immunglobulins ausgetauscht wurde;
wobei jede Teilbibliothek, die Nucleinsäuren enthält, die für mutierte Immunglobuline codieren, Nucleinsäuren umfasst, die für mutierte Immunglobuline codieren, in denen die einzelne vorher bestimmte Aminosäure wenigstens einmal in jeder Position in der Komplementarität-bestimmenden Region, in die die einzelne vorher bestimmte Aminosäure eingeführt wurde, vorhanden ist.

3. Bibliothek gemäß Anspruch 1 oder Anspruch 2, wobei das Immunglobulin von Interesse ein katalytischer Antikörper, IgG, IgM, IgA, IgD, IgE, ein Fab-Fragment eines Immunglobulins oder ein Einzelketten-Immunglobulin ist.

4. Universalbibliothek, unfassend:
20 einzelne vorher bestimmte Aminosäure-Bibliotheken, bestehend aus einer einzelnen vorbestimmten Aminosäure-Bibliothek gemäß Anspruch 1, 2 oder 3 für jede der 20 natürlich vorkommenden Aminosäuren.

## Revendications

1. Banque d'acides aminés prédéterminés individuels comprenant des immunoglobulines d'intérêt ayant six régions déterminant la complémentarité, la banque incluant des sous-banques comprenant :
a) une sous-banque comprenant une immunoglobuline prototype d'intérêt d'après laquelle toutes les mutations suivantes sont basées ; et
b) six sous-banques comprenant chacune des immunoglobulines mutées dans lesquelles un acide aminé prédéterminé individuel a été substitué en une ou plusieurs positions dans l'une des six régions déterminant la complémentarité de l'immunoglobuline prototype, avec une sous-banque pour chacune des six régions déterminant la complémentarité ; et
c) 15 sous-banques comprenant chacune des immunoglobulines mutées dans lesquelles l'acide aminé prédéterminé individuel a été substitué en une ou plusieurs positions dans deux des six régions déterminant la complémentarité de l'immunoglobuline prototype, avec une sous-banque pour chacune des combinaisons possibles de deux des six régions déterminant la complémentarité ; et
d) 20 sous-banques comprenant chacune des immunoglobulines mutées dans lesquelles l'acide aminé prédéterminé individuel a été substitué en une ou plusieurs positions dans trois des six régions déterminant la complémentarité de l'immunoglobuline prototype, avec une sous-banque pour chacune des combinaisons possibles de trois des six régions déterminant la complémentarité ; et
e) 15 sous-banques comprenant chacune des immunoglobulines mutées dans lesquelles l'acide aminé prédéterminé individuel a été substitué en une ou plusieurs positions dans quatre des six régions déterminant la complémentarité de l'immunoglobuline prototype, avec une sous-banque pour chacune des combinaisons possibles de quatre des six régions déterminant la complémentarité ; et
f) 6 sous-banques comprenant chacune des immunoglobulines mutées dans lesquelles l'acide aminé prédéterminé individuel a été substitué en une ou plusieurs positions dans cinq des six régions déterminant la complémentarité de l'immunoglobuline prototype, avec une sous-banque pour chacune des combinaisons possibles de cinq des six régions déterminant la complémentarité ; et
g) une sous-banque comprenant des immunoglobulines mutées dans lesquelles l'acide aminé prédéterminé individuel a été substitué en une ou plusieurs positions dans les six régions déterminant la complémentarité de l'immunoglobuline prototype ;
banque dans laquelle chaque sous-banque contenant des immunoglobulines mutées comprend des immunoglobulines mutées dans lesquelles l'acide aminé prédéterminé individuel est présent au moins une fois en chaque position dans la région déterminant la complémentarité dans laquelle l'acide aminé prédéterminé individuel a été introduit.

2. Banque d'acides aminés prédéterminés individuels comprenant des acides nucléiques codant pour des immunoglobulines d'intérêt ayant six régions déterminant la complémentarité, la banque incluant des sous-banques comprenant :
a) une sous-banque comprenant des acides nucléiques codant pour une immunoglobuline prototype d'intérêt d'après laquelle toutes les mutations suivantes sont basées ; et
b) six sous-banques comprenant chacune des acides nucléiques codant pour des immunoglobulines mutées dans lesquelles un acide aminé prédéterminé individuel a été substitué en une ou plusieurs positions dans l'une des six régions déterminant la complémentarité de l'immunoglobuline prototype, avec une sous-banque pour chacune des six régions déterminant la complémentarité ; et
c) 15 sous-banques comprenant chacune des acides nucléiques codant pour des immunoglobulines mutées dans lesquelles l'acide aminé prédéterminé individuel a été substitué en une ou plusieurs positions dans deux des six régions déterminant la complémentarité de l'immunoglobuline prototype, avec une sous-banque pour chacune des combinaisons possibles de deux des six régions déterminant la complémentarité ; et
d) 20 sous-banques comprenant des acides nucléiques codant pour des immunoglobulines mutées dans lesquelles l'acide aminé prédéterminé individuel a été substitué en une ou plusieurs positions dans trois des six régions déterminant la complémentarité de l'immunoglobuline prototype, avec une sous-banque pour chacune des combinaisons possibles de trois des six régions déterminant la complémentarité ; et
e) 15 sous-banques comprenant chacune des acides nucléiques codant pour des immunoglobulines mutées dans lesquelles l'acide aminé prédéterminé individuel a été substitué en une ou plusieurs positions dans quatre des six régions déterminant la complémentarité de l'immunoglobuline prototype, avec une sous-banque pour chacune des combinaisons possibles de quatre des six régions déterminant la complémentarité ; et
f) 6 sous-banques comprenant chacune des acides nucléiques codant pour des immunoglobulines mutées dans lesquelles l'acide aminé prédéterminé individuel a été substitué en une ou plusieurs positions dans cinq des six régions déterminant la complémentarité de l'immunoglobuline prototype, avec une sous-banque pour chacune des combinaisons possibles de cinq des six régions déterminant la complémentarité ; et
g) une sous-banque comprenant des acides nucléiques codant pour des immunoglobulines mutées dans lesquelles l'acide aminé prédéterminé individuel a été substitué en une ou plusieurs positions dans les six régions déterminant la complémentarité de l'immunoglobuline prototype ;
banque dans laquelle chaque sous-banque contenant des acides nucléiques codant pour des immunoglobulines mutées comprend des acides nucléiques codant pour des immunoglobulines mutées dans lesquelles l'acide aminé prédéterminé individuel est présent au moins une fois en chaque position dans la région déterminant la complémentarité dans laquelle l'acide aminé prédéterminé individuel a été introduit.

3. Banque selon la revendication 1 ou la revendication 2, dans laquelle l'immunoglobuline d'intérêt est un anticorps catalytique ; une IgG ; une IgM ; une IgA ; une IgD ; une IgE ; un fragment Fab d'une immunoglobuline ; ou une immunoglobuline à chaîne unique.

4. Banque universelle comprenant :
vingt banques d'acides aminés prédéterminés individuels consistant en une banque d'acides aminés prédéterminés individuels selon la revendication 1, 2 ou 3 pour chacun des vingt acides aminés naturels.
